# EUROPÄISCHE PATENTSCHRIFT

(11) **EP 0 866 065 B1**
(45) Veröffentlichungstag und Bekanntmachung des Hinweises auf die Patenterteilung: **13.04.2005**
(21) Anmeldenummer: 98102136.3
(22) Anmeldetag: 07.02.1998
(51) Int. Cl.: C07D 319/06, C07D 319/08

(54) **Verfahren zur Herstellung von 1,3-Dioxanverbindungen**
Process for the preparation of 1,3-dioxane compounds
Procédé pour la préparation des composés de 1,3-dioxane

(30) Priorität: 21.03.1997 DE 19711758
(43) Veröffentlichungstag der Anmeldung: 23.09.1998
(73) Patentinhaber: Degussa AG, 40474 Düsseldorf (DE)
(72) Erfinder: Bauer, Frank, Dr., 53127 Bonn (DE); Neumann, Manfred, Dr., 45770 Marl (DE)

(56) Entgegenhaltungen:
- EP-A- 0 220 034
- EP-A- 0 268 460
- DE-A- 4 040 685
- S. MAGER ET AL.: STUDIA UNIVERSITATIS BABES-BOLYAI - CHEMIA, Bd. 24, Nr. 1, 1979, Seiten 32-38, XP002071034
- E. L. ELIEL ET AL.: JOURNAL OF THE AMERICAN CHEMICAL SOCIETY, Bd. 94, Nr. 1, 12.Januar 1972, Seiten 171-6, XP002071035
- K. N. WELCH: JOURNAL OF THE CHEMICAL SOCIETY, 1930, LONDON, Seiten 257-61, XP002071036

## Beschreibung

Die vorliegende Erfindung betrifft ein Verfahren zur Herstellung von 1,3-Dioxanverbindungen der allgemeinen Formel 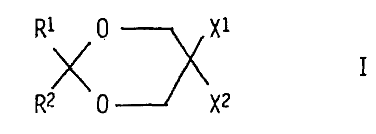 in der R¹ und R² unabhängig voneinander Wasserstoff oder einen Kohlenwasserstoffrest bedeuten und X¹ und X² gleiche oder verschiedene elektronenanziehende Gruppen bedeuten, mit den für R¹, R², X¹ und X² jeweils weiter unten angegebenen Definitionen.

1,3-Dioxanverbindungen der allgemeinen Formel I sind als organische Zwischenprodukte, z.B. als Vorstufen für UV-Stabilisatoren oder Röntgenkontrastmittel, von kommerziellem Interesse (siehe z.B. EP-A2 0 220 034)

Üblicherweise werden 1,3-Dioxanverbindungen der allgemeinen Formel I aus den entsprechenden Bishydroxymethylverbindungen II durch säurekatalysierte Acetalisierung bzw. Ketalisierung gemäß der Reaktionsgleichung 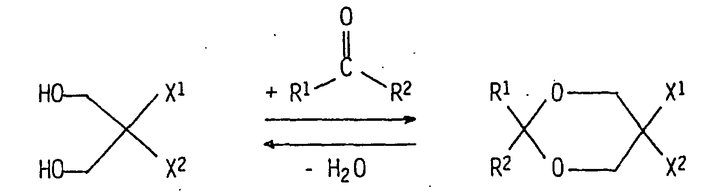 hergestellt (siehe z.B. Mager. S.; Hopartean, I; Horn, M.; Groso. I.; Stud. Univ., Babes-Bolyai, [Ser.] Chem. 1979, 24(1), 23-8). Die Verschiebung des Gleichgewichts erfolgt dabei durch Azeotropdestillation unter Einsatz eines geeigneten Schleppmittels, wie Toluol oder Cyclohexan.

Eine Übertragung dieses Verfahrens in den technischen Maßstab bereitet jedoch Schwierigkeiten, welche vorwiegend aus der thermischen Empfindlichkeit der Bishydroxymethylverbindungen resultieren. So zersetzt sich der technisch bedeutsame Bishydroxymethylmalonsäurediethylester bereits bei Temperaturen oberhalb von 50°C zu einer Vielzahl von Folgeprodukten, unter anderem zu Formaldehyd (Welch, K. N.; J. Chem. Soc. London, 1930, 1). Bei Cyanogruppen enthaltenden Bishydroxymethylverbindungen besteht die Gefahr, daß sich toxischer Cyanwasserstoff bildet.

Erwartungsgemäß wurden daher unter Einstellung technisch relevanter Verweilzeiten selbst bei Verwendung von niedrigsiedenden Schleppmitteln, wie Toluol, Cyclohexan oder Isopropylacetat, je nach dem verwendeten Katalysator Produktverluste von bis zu 50% d.Th. beobachtet.

So werden in EP 268 460 Beispiele zur Acetalisierung von isolierten Bishydroxymethylmalonsäureestem mit verschiedenen Aldehyden gegeben, bei denen Benzol oder Toluol als Schleppmittel eingesetzt werden, wobei man Ausbeuten von 16,3 bis 74,1 % der Theorie erhält. In DE 4 040 685 wird Bishydroxymethylmalonsäurediethylester mit Benzaldehyd in Toluol acetalisiert und dabei eine Ausbeute von 78 % der Theorie nach Destillation erhalten. Das Toluol wurde vor der Destillation im Vakuum abgezogen.

Eine Begrenzung der Sumpftemperatur auf 50°C und mithin eine Vermeidung von thermischen Zersetzungsreaktionen kann im Labormaßstab auf einfache Weise durch Anlegen von Vakuum erreicht werden. In diesem Falle liefert etwa die Umsetzung von Bishydroxymethylmalonsäurediethylester mit einer äquimolaren Menge an Cyclohexanon in Toluol unter Schwefelsäurekatalyse eine isolierte Ausbeute von >85 % d.Th.. Im technischen Maßstab ist aber die Kondensation der Brüden mit sehr hohen Investitions- und Betriebskosten verbunden.

Eine thermische Zersetzung der Bishydoxymethylverbindungen unter den Reaktionsbedingungen der Acetalisierung bzw. Ketalisierung kann auch durch Verwendung von niedrigsiedenden Schleppmitteln verhindert werden (Eliel, E. L.; Banks. H. D.; J. Am. Chem. Soc. 94 (1972), 171). Es wurde jedoch gefunden, daß niedrigsiedende Schleppmittel, wie Petrolether (30 - 60°C), Methyl-tert.-butylether oder Methylacetat, das oben dargestellte Gleichgewicht nur sehr langsam - und im Falle von sterisch gehinderten Ketonen auch nur unvollständig - verschieben. Die erzielbaren Raum-Zeit-Ausbeuten sind daher für technische Realisierungen völlig unzureichend. Gegen eine Realisierung dieser Vorgehensweise im technischen Maßstab sprechen darüber hinaus die hohe Feuergefährlichkeit niedrigsiedender Petrolether (Flammpunkt < 20°C) sowie die üblicherweise geringen Löslichkeiten der stark polaren Bishydroxymethylverbindungen.

Es bestand also die Aufgabe, ein Verfahren bereitzustellen, das eine möglichst schnelle und vollständige Umsetzung der Bishydroxymethylverbindungen zu den Verbindungen der allgemeinen Formel I unter Bedingungen gestattet, unter denen Produktverluste durch thermische Zersetzung der Edukte praktisch ausgeschlossen sind.

Es wurde gefunden, daß diese Aufgabe auf einfache Weise gelöst wird und man 1,3-Dioxanverbindungen der allgemeinen Formel 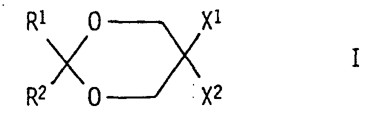 in der R¹ und R² jeweils unabhängig voneinander Wasserstoff, Alkyl, Aralkyl, Aryl oder Cycloalkyl mit jeweils bis zu 12 Kohlenstoffatomen bedeuten und zudem gemeinsam einen Alkylenrest mit 4 bis 11 Kohlenstoffatomen bezeichnen können und X¹ und X² jeweils unabhängig voneinander COOR, CONR₂, CN, NO₂, C(OR)=NR₂ oder COR bedeuten, wobei R für Wasserstoff, Alkyl, Aralkyl, Aryl oder Cycloalkyl mit jeweils bis zu 12 Kohlenstoffatomen steht; mit der Maßgabe, daß (i) nicht beide Substituenten X¹ und X² gleichzeitig COOH bedeuten können und (ii), wenn beide Substituenten X¹ und X² gleichzeitig COR bedeuten, die beiden Substituenten R auch einen Alkylenrest mit 2 bis 9 Kohlenstoffatomen bedeuten können, vorteilhaft erhält, wenn man eine Bishydroxymethylverbindung der allgemeinen Formel 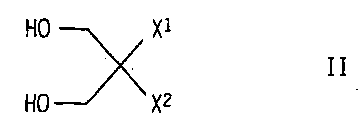 in der X¹ und X² jeweils unabhängig voneinander die angegebene Bedeutung haben, mit einem Orthocarbonsäureester der allgemeinen Formel

R⁴-C(OR⁵)₃ III

in der R⁴ Wasserstoff oder einen Kohlenwasserstoffrest und R⁵ einen Kohlenwasserstoffrest bedeuten, und mit einem Aldehyd oder Keton der allgemeinen Formel

R¹-CO-R² IV

umsetzt, in der R¹ und R² jeweils unabhängig voneinander die zuvor angegebene Bedeutung haben.

Unter den sehr schonenden Bedingungen des erfindungsgemäßen Verfahrens dürfte als erste Teilreaktion zunächst eine sehr schnell verlaufende Umsetzung der Bishydroxymethylverbindungen II zu Verbindungen der allgemeinen Formel 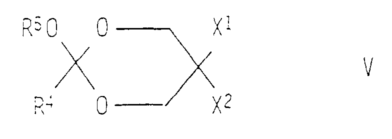 erfolgen, in der R⁴, R⁵, X¹ und X² die angegebenen Bedeutung haben. Dadurch wird die empfindliche Bishydroxymethylverbindung II rasch den Bedingungen entzogen, unter denen sie zur Zersetzung neigt. Die Verbindung V, ein cyclischer Orthocarbonsäureester, ist unter diesen Bedingungen stabil und reagiert in einer zweiten Teilreaktion mit dem Aldehyd oder Keton der allgemeinen Formel IV zu der 1,3-Dioxanverbindung der allgemeinen Formel I, einem cyclischen Acetal oder Ketal.

Bei einer zweckmäßigen Ausführungsform des erfindungsgemäßen Verfahrens wird die Bishydroxymethylverbindung II nacheinander mit dem Orthocarbonsäureester III und dem Aldehyd oder Keton IV umgesetzt. Dem obigen Ablauf in zwei Teilschritten entsprechend ist es vorteilhaft. der Bishydroxymethylverbindung der allgemeinen Formel II zunächst den Orthocarbonsäureester der allgemeinen Formel III zuzusetzen und dann dem Reaktionsgemisch den Aldehyd oder das Keton der allgemeinen Formel IV zuzufügen. Im Prinzip ist jedoch die Reihenfolge der Zugabe der Reaktanten III und IV beliebig. So kann man die beiden Reaktanten gleichzeitig oder zeitüberlappend zuführen. Wegen der stark unterschiedlichen Reaktionsgeschwindigkeiten der erwähnten Teilreaktionen ist es sogar möglich, den Orthocarbonsäureester der allgemeinen Formel III erst dann zuzugeben, wenn die gesamte Menge des Aldehyds oder Ketons der allgemeinen Formel IV zugeführt wurde. Auch dabei werden Ausbeuten und Raum-Zeit-Ausbeuten erzielt, die denjenigen des Standes der Technik überlegen sind.

Im Falle der Umsetzung von Bishydroxymethymalonsäurediethylester mit Triethylorthoformiat und Aceton wird das Verfahren nach der

Erfindung durch das folgende Reaktionsschema wiedergegeben:

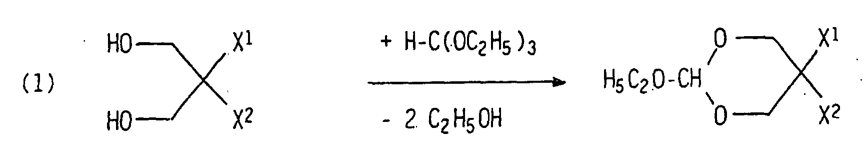

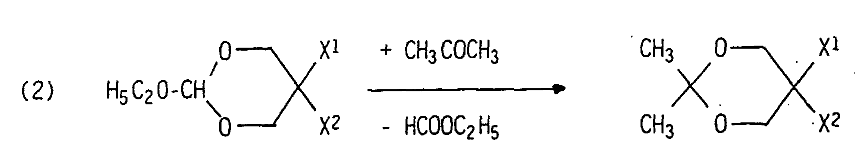

In bevorzugten 1,3-Dioxanverbindungen der allgemeinen Formel I bedeuten X¹ und X² jeweils unabhängig voneinander CN oder COOR', wobei R' einen C₁₋₄-Alkylrest bezeichnet.

In den erfindungsgemäßen Bishydroxymethylverbindungen der allgemeinen Formel II haben X¹ und X² jeweils unabhängig die für die erfindungsgemäßen 1,3-Dioxanverbindungen der allgemeinen Formel I angegebenen Bedeutungen. Von den erfindungsgemäßen Bishydroxymethylverbindungen seien z.B.

Bishydroxymethylmalonsäuredimethylester, Bishydroxymethylmalonsäurediethylester, Bishydroxymethylmalonsäuredi-n-propylester, Bishydroxymethylmalonsäurediisobutylester, Bishydroxymethylmalonsäuredibenzylester, Bishydroxymethylmalonsäuredi-2-ethylhexylester, Bishydroxymethyl-N,N-dimethylcarbamidoessigsäureethylester, Bishydroxymethyl-N,N,N',N'-tetramethylmalonsäurediamid, Bishydroxymethylmalonsäuredinitril, Bishydroxymethylcyanessigsäureethylester, Bishydroxymethylcyanessigsäure-n-butylester, Bishydroxymethylnitroessigsäureethylester, 3,3-Bishydroxymethylacetylaceton und 2,2-Bishydroxymethylcyclododeca-1,3-dion genannt. In den bevorzugten Bishydroxymethylverbindungen II haben X¹ und X² die für die bevorzugten 1,3-Dioxanverbindungen angegebene Bedeutung, d. h. CN oder COOR'.

Bevorzugte Orthocarbonsäureester der allgemeinen Formel III sind die Orthoameisensäureester (R⁴ = H) von Alkanolen mit 1 bis 4 Kohlenstoffatomen (R⁵ = C₁₋₄-Alkyl). Beispiele hierfür sind Triethylorthoformiat, Triisobutylorthoformiat und, besonders bevorzugt, Trimethylorthoformiat. Wenn in der Bishydroxymethylverbindung der allgemeinen Formel II die Substituenten X¹ und/oder X² -COOR bedeuten und R von R⁴ und/oder R⁵ in der allgemeinen Formel III verschieden sind, könnte eine Umesterung stattfinden. Bemerkenswerterweise ist das nicht der Fall, so daß man in der Auswahl der Orthocarbonsäureester frei ist. Man setzt den Orthocarbonsäureester zweckmäßig in mindestens stöchiometrischer Menge ein. Im Überschuß verwendet dient er zugleich als Lösungsmittel. Gegebenenfalls empfiehlt sich auch die Mitverwendung eines inerten Lösungsmittels, z.B. eines Alkanols, wie in der Folge näher erläutert wird.

In den erfindungsgemäßen Aldehyden oder Ketonen der allgemeinen Formel IV haben R¹ und R² unabhängig voneinander die für die erfindungsgemäßen 1,3-Dioxan-Verbindungen der allgemeinen Formel I angegebene Bedeutung. Von den erfindungsgemäßen Aldehyden und Ketonen seien z. B. Formaldehyd,

Acetaldehyd, n- und iso-Butyraldehyd, Benzaldehyd. Phenylacetaldehyd, Aceton, Methylethylketon, Diisopropylketon, Cyclohexanon und Cyclododecanon genannt. Der Aldehyd oder das Keton IV wird zweckmäßig ebenfalls in stöchiometrischer Menge oder in einem Überschuß, z.B. von bis zu 200 %, eingesetzt. Bei noch größeren Überschüssen leidet die Raum-Zeit-Ausbeute.

Das erfindungsgemäße Verfahren läßt sich schon unterhalb von Raumtemperatur durchführen. Oberhalb von 40°C ist die Umsetzung meist innerhalb weniger Stunden beendet. Besonders bevorzugt wird daher bei absatzweiser Durchführung eine Temperatur im Bereich von 0°C bis 80°C, insbesondere von 20 bis 50°C. In der Praxis hat es sich als vorteilhaft erwiesen, die Temperatur gegen Ende der Umsetzung zu steigern, z.B. um etwa 10°C bis 30°C. Die Reaktionstemperatur und die Reaktionszeit sollten so aufeinander abgestimmt werden, daß Zersetzungsreaktionen praktisch ausgeschlossen werden. Zweckmäßig wählt man eine solche Reaktionstemperatur, daß während der Reaktion gebildete Leichtsieder, wie Alkanole und Alkylformiate, aus dem Reaktionsgemisch abdestillieren. Wenn die Verweilzeiten durch geeignete Maßnahmen, insbesondere durch eine kontinuierliche Reaktionsführung, ausreichend verkürzt werden, so kann das Verfahren auch bei weit höheren Temperaturen, wie 200°C und höher, mit entsprechend kurzen Reaktionszeiten durchgeführt werden.

Durch geeignete Wahl von Reaktionstemperatur und Reaktionszeit kann bei dem erfindungsgemäßen Verfahren auch unter technischen Bedingungen eine Zersetzung der Bishydroxymethylverbindung der allgemeinen Formel II weitgehend zurückgedrängt werden. Die Ausbeuten an 1,3-Dioxanverbindungen der allgemeinen Formel I sind dementsprechend höher als die nach der "Azeotropmethode" erzielten Ausbeuten. So wurden bei der Herstellung von 2-Isopropyl-1,3-dioxan-5,5-dicarbonsäurediethylester nach dem erfindungsgemäßen Verfahren Ausbeuten von bis zu 82 % d.Th. erzielt. Dieser Wert steht einer Ausbeute von 77 % d.Th. gegenüber (Eliel et al., loc. cit.), die noch dazu durch Azeotropdestillation mit Petrolether (30 bis 60°C) als Schleppmittel und somit auf einem für technische Anwendungen nicht geeigneten Weg erzielt wurde. Höhersiedende Schleppmittel führten zu noch niedrigeren Ausbeuten an dem Zielprodukt (Eliel et. al., loc. cit.).

Die Bishydroxymethylverbindungen der allgemeinen Formel II können in reiner Form, aber auch in Form von Lösungen in inerten Losungsmitteln, vorteilhaft in Alkoholen, wie Ethanol, eingesetzt werden. Auf jeden Fall sollte die Bishydroxymethylverbindung oder ihre Lösung möglichst wasserfrei sein. Anderenfalls kann dem Wassergehalt durch eine entsprechende Aufstockung der Menge an Orthocarbonsäureester der allgemeinen Formel III Rechnung getragen werden.

Besonders vorteilhaft wird das erfindungsgemäße Verfahren so ausgeführt, daß die Bishydroxymethylverbindung der allgemeinen Formel II durch Umsetzung von C-H-aciden Verbindungen der allgemeinen Formel 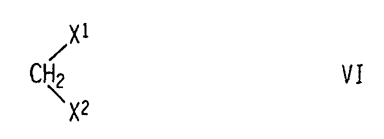 in der X¹ und X² die angegebene Bedeutung haben, mit Formaldehyd oder einer Formaldehyd abgebenden Verbindung, gegebenenfalls in einem inerten Lösungsmittel, hergestellt und ohne Isolierung im Reaktionsgemisch umgesetzt wird. Vorteilhaft verwendet man einen Orthocarbonsäureester der allgemeinen Formel III (oder ein Gemisch solcher Ester) als Lösungsmittel und arbeitet zweckmäßig nach dem Verfahren der gleichzeitig anhängigen Patentanmeldung DE 197 11 762 (O.Z. 5172). Der Reaktant Orthocarbonsäureester III dient in dieser Vorstufe als Lösungsmittel, so daß auf ein weiteres inertes Lösungsmittel ganz oder teilweise verzichtet werden kann. Für die erfindungsgemäße Reaktion fungiert der Orthocarbonsäureester dann als Reaktant III. Dieses "Eintopfverfahren" stellt eine Vereinfachung der erfindungsgemäßen Arbeitsweise dar, vermindert den apparativen Aufwand und optimiert die Raum-Zeit-Ausbeute, bezogen auf die C-Hacide Verbindung der allgemeinen Formel VI.

Die Umsetzung der Bishydroxymethylverbindungen der allgemeinen Formel II mit dem Aldehyd oder Keton IV und dem Orthoester III nach dem erfindungsgemäßen Verfahren wird durch saure Katalysatoren gefördert. Man setzt daher zweckmäßig eine starke Mineralsäure, wie Chlorwasserstoff oder Schwefelsäure, oder saure Salze der letzteren, wie ein, Andere geeignete Katalysatoren sind saure Festbettkatalysatoren, z.B. Ionenaustauscher auf organischer Basis, wie Sulfosäuregruppen enthaltende Phenol-Formaldehyd-Harze, oder auf anorganischer Basis, wie saure Montmorillonite. Man verwendet die sauren Katalysatoren im allgemeinen in Mengen von 0,05 bis 5,0 Gewichtsprozent, vorzugsweise von 0,05 bis 2,0 Gewichtsprozent, bezogen auf das Reaktionsgemisch. Saure Ionenaustauscher werden zweckmäßig in Mengen von 2 bis 10 Gewichtsprozent eingesetzt. Deren Anteil beträgt noch mehr, wenn man das Ausgangsgemisch über fest angeordnete saure Ionenaustauscher laufen läßt.

Als besonders geeignete Katalysatoren haben sich Alkalihydrogensulfate, wie Natriumhydrogensulfat, erwiesen, allein oder zusammen mit Schwefelsäure. Auch ohne Neutralisation des Reaktionsgemisches treten dann bei der destillativen Isolierung der 1,3-Dioxanverbindungen der allgemeinen Formel I praktisch keine Zersetzungen auf. Aber auch bei Verwendung anderer saurer Katalysatoren kann auf die sonst notwendige - und unter technischen Bedingung sehr aufwendige - wäßrige Aufarbeitung des Reaktionsgemisches verzichtet werden, wenn der Katalysator, z.B. durch Filtration oder Neutralisation mir einer Base, wie Natriumhydroxid, -alkoholat, -carbonat oder -hydrogencarbonat, neutralisiert wird.

Die 1,3-Dioxanverbindungen der allgemeinen Formel I können durch fraktionierte Destillation des von Leichtsiedern befreiten Reaktionsgemisches isoliert werden, sofern sie nicht im Reaktionsgemisch weiter umgesetzt werden. Letzteres empfiehlt sich besonders bei 1,3-Dioxanverbindungen, die sich von langkettigen Aldehyden oder Ketonen ableiten.

Die folgenden Beispiele sollen das Verfahren nach der Erfindung weiter erläutern, nicht aber dessen Anwendungsbereich limitieren.

### Beispiel 1 (Vergleichsbeispiel)

### 1,5-Dioxaspiro[5,5]undecan-3,3-dicarbonsäurediethylester

Zu einer gerührten Mischung aus 165,0 g Bishydroxymethylmalonsäurediethylester (98-%ig, 0,75 Mol), 75,3 g Cyclohexanon (0,75 Mol) und 500 g Cyclohexan als Lösungs- und Schleppmittel wurde 1,0 g Schwefelsäure gegeben. Anschließend erhitzte man die Mischung auf Rückflußtemperatur (70-80°C) und kreiste das gebildete Reaktionswasser innerhalb von 5 Stunden aus.

Nach beendeter Reaktion wurde das Reaktionsgemisch auf Raumtemperatur abgekühlt und in verdünnte, überschüssige wäßrige Natriumhydrogencarbonatlösung eingetragen. Die wäßrige Phase wurde mit Methyltert.-butylether nachextrahiert, und die vereinigten organischen Phase wurden einmal mit Wasser gewaschen. Nach Trocknen über Natriumsulfat wurden die Lösungsmittel am Rotationsverdampfer abdestilliert, und das Produkt wurde durch Destillation im Ölpumpenvakuum isoliert. Man erhielt 168,4 g Zielprodukt (75% d.Th., bezogen auf eingesetzten Bishydroxymethylmalonsäurediethylester) vom Siedepunkt 140°C/0,2 mm. Die gaschromatographisch bestimmte Reinheit betrug 97 bis 98 FID-Flächenprozent.

### Beispiel 2

### 1,5-Dioxaspiro[5,5]undecan-3,3-dicarbonsäurediethylester

Zu einer bei Raumtemperatur gerührten Suspension aus 60,0 g p-Formaldehyd (2,0 Mol) und 0,25 g Natriummethylat in 40,0 g Ethanol wurden innerhalb von 1,75 Stunden 160,2 g Diethylmalonat (1,0 Mol) zudosiert, wobei die Innentemperatur durch Kühlen zwischen 20°C und 30°C gehalten wurde. Anschließend ließ man 2 Stunden bei 50°C nachreagieren, bevor 148.2 g Trimethylorthoformiat zudosiert wurden. Die Mischung wurde nach Zugabe von 0,4 g Schwefelsäure weitere 2,5 Stunden auf 50°C erwärmt, wobei ein schwacher Rückfluß zu beobachten war. Bei einer Sumpftemperatur von bis zu 80°C wurden dann 129,0 g Destillat vom Siedebereich 30°C bis 55°C abgenommen, dann dosierte man innerhalb von 1,45 Stunden 98,0 g Cyclohexanon (1,0 Mol) zu, wobei weitere 25,9 g Leichtsieder vom Siedebereich 36°C bis 44°C abdestilliert wurden.

Nach Abkühlen auf Raumtemperatur wurde die Reaktionsmischung durch Einrühren in 600 ml Natriumhydrogencarbonatlösung. Abtrennen der organischen Phase und zweimalige Extraktion der wäßrigen Phase mit jeweils 200 ml Methyl-tert.-butylether wäßrig aufgearbeitet. Die anschließende fraktionierte Destillation ohne Verwendung einer Kolonne lieferte 197,8 g farbloses Zielprodukt (63,3 % d.Th.. bezogen auf eingesetztes Diethylmalonat) mit einer gaschromatographisch bestimmten Reinheit von 95 FID-Flächenprozent.

### Beispiel 3

### 1,5-Dioxaspiro[5,5]undecan-3,3-dicarbonsäurediethylester

Zu einer bei Raumtemperatur gerührten Suspension aus 148,4 g Trimethylorthoformiat (1,4 Mol) und 63,0 g p-Formaldehyd (2,1 Mol) wurden 10,0 g Ethanol und 0,25 g Natriumethanolat gegeben. Anschließend wurde auf 50°C aufgeheizt, und man dosierte innerhalb von 1.75 Stunden 160,2 g Diethylmalonat (1,0 Mol) hinzu. Die so erhaltene Reaktionsmischung wurde zunächst noch 2 Stunden bei 50°C geruhrt. dann auf Raumtemperatur abgekühlt. Man fügte unter Rühren 0,37 g Schwefelsäure und 1,0 g Natriumhydrogensulfat zu, bevor zunächst weitere 2 Stunden auf 50°C temperiert wurde. Beim folgenden Erhitzen auf 80°C wurden 72,3 g Leichtsieder abgenommen, dann dosierte man innerhalb von 1,75 Stunden 98,0 g Cyclohexanon (1,0 Mol) zu, wobei weitere 74,4 g Leichtsieder übergingen. Die anschließende fraktionierte Destillation ohne Verwendung einer Kolonne lieferte 246,2 g farbloses Zielprodukt (78,3 % d.Th., bezogen auf eingesetztes Diethylmalonat) mit einer gaschromatographisch bestimmten Reinheit von 95,4 FID-Flächenprozent.

### Beispiel 4

### 1,5-Dioxaspiro[5,5]undecan-3,3-dicarbonsäurediethylester

Man verfuhr wie in Beispiel 3, jedoch erfolgte vor der destillativen Isolierung des Produktes eine wäßrige Aufarbeitung unter Verwendung von gesättigter Natriumhydrogencarbonatlösung. Durch anschließende fraktionierte Destillation ohne Verwendung einer Kolonne konnten 253,0 g farbloses Zielprodukt (80 % d.Th., bezogen auf eingesetztes Diethylmalonat) mit einer gaschromatographisch bestimmten Reinheit von 96 FID-Flächenprozent erhalten werden.

### Beispiel 5

### 1,5-Dioxaspiro[5,5]undecan-3,3-dicarbonsäurediethylester

Zu einer bei Raumtemperatur gerührten Suspension aus 60,0 g p-Formaldehyd (2,0 Mol) und 0,25 g Natriumethylat in 50,0 g Ethanol wurden innerhalb von einer Stunde 160,2 g Diethylmalonat (1,0 Mol) zudosiert, wobei die Innentemperatur durch Kühlen zwischen 20°C und 30°C gehalten wurde. Anschließend ließ man 2 Stunden bei 50°C nachreagieren, bevor 0,4 g Schwefelsäure und 1,0 g Natriumhydrogensul-fat zugegeben und 98 g Cyclohexanon (1,0 Mol) innerhalb von 5 Minuten zudosiert wurden. Man erwärmte die Mischung eine Stunde lang auf 50°C, dosierte innerhalb von einer Stunde 112,2 g Trimethylorthoformiat zu und ließ dann noch weitere 2 Stunden bei 50°C nachreagieren. Beim folgenden Erhitzen gingen 159,6 g Leichtsieder über. Der verbliebene Rückstand wurde im Ölpumpenvakuum fraktioniert, wobei man 213,8 g Zielprodukt Kolonne lieferte 197,8 g farbloses Zielprodukt (74,1 % d.Th., bezogen auf eingesetztes Diethylmalonat) erhielt. Die gaschromatographisch bestimmte Reinheit betrug 96 FID-Flächenprozent.

### Beispiel 6

### 2-Isopropyl-1,3-dioxan-5,5-dicarbonsäurediethylester

Eine Mischung aus 165,0 g Bishydroxymethylmalonsäurediethylester, 83,4 g Trimethylorthoformiat und 0,6 g Natriumhydrogensulfat wurde unter Rühren 30 Minuten lang auf 50°C und anschließend 90 Minuten lang auf 60°C erhitzt. Dann dosierte man innerhalb von 45 Minuten 81,0 g Isobutyraldehyd zu, wobei die Innentemperatur durch leichtes Kühlen auf 60°C gehalten wurde. Die gebildeten Leichtsieder wurden bis zu einer Sumpftemperatur von 120°C abdestilliert, dann gab man zur Neutralisation des Katalysators 1,0 g Natriumcarbonat zu. Durch anschließende Destillation im Ölpumpenvakuum wurden 168,2 g (82 % d.Th., bezogen auf eingesetzten Bishydroxymethylmalonsäurediethylester) farbloses Zielprodukt vom Siedepunkt 110°C/1 mm erhalten. Die gaschromatographisch bestimmte Reinheit des Produktes betrug 98 FID-Flächenprozent.

### Beispiel 7

### 2-Isopropyl-1,3-dioxan-5,5-dicarbonsäurediethylester

Man verfuhr wie in Beispiel 6, jedoch wurde auf den Zusatz von Natriumcarbonat vor der destillativen Produktisolierung verzichtet. Die Ausbeute an farblosem Zielprodukt betrug 164,6 g (80 % d.Th., bezogen auf eingesetzten Bishydroxymethylmalonsäurediethylester). Das Produkt wies eine gaschromatographisch bestimmte Reinheit von 98 FID-Flächenprozent auf.

### Beispiel 8

### 2-Isopropyl-1,3-dioxan-5,5-dicarbonsäurediethylester

Zu einer bei Raumtemperatur gerührten Suspension aus 30,0 g p-Formaldehyd (1,0 Mol) in 25,0 g Ethanol wurden 0,13 g Natriumethanolat gegeben. Die Mischung wurde 20 Minuten gerührt, dann dosierte man innerhalb von 1,5 Stunden 80,1 g Diethylmalonat (0,5 Mol) zu, wobei die Innentemperatur zwischen 26°C und 28°C gehalten wurde. Die so erhaltene Reaktionsmischung wurde noch 2 Stunden bei 50°C gerührt, dann auf Raumtemperatur abgekühlt. Man fügte unter Rühren 0,18 g konzentrierte Schwefelsäure. 55,7 g Trimethylorthoformiat (0,53 Mol) und 1,5 g Natriumhydrogensulfat-Monohydrat zu, bevor zunächst weitere 3,0 Stunden auf 50°C temperiert wurde.

Anschließend wurden innerhalb von 2 Stunden 54,0 g Isobutyraldehyd (0,75 Mol) zudosiert, wobei unter Einsatz einer 20-cm-Füllkörperkolonne und bei Sumpftemperaturen zwischen 60°C und 80°C insgesamt 81,3 g Leichtsieder vom Siedebereich 23°C bis 68°C abgenommen wurden. Nachdem durch Steigern der Sumpftemperatur auf 120°C weitere 19.1 g Leichtsieder abdestilliert wurden, fraktionierte man den verbliebenen Rückstand ohne Verwendung einer Kolonne im Öl^{p}umpenvakuum. Hierbei wurden 97,6 g farbloses Zielprodukt (69,8 % d.Th., bezogen auf eingesetztes Diethylmalonat) mit einer gaschromatographisch bestimmten Reinheit von 98 FID-Flächenprozent erhalten.

### Beispiel 9

### 1,5-Dioxaspiro[5,5]undecan-3-carbonsäureethylester-3-cyano

Zu einer bei 0°C bis 10°C gerührten Mischung aus 62,0 g p-Formaldehyd (2,1 Mol), 200 g Ethanol und 0,25 g Natriumethanolat wurden innerhalb von 2,0 Stunden 113,0 g Cyanessigsäureethylester (1,0 Mol) dosiert. Die Reaktionsmischung wurde anschließend noch 1 Stunde bei 10°C gerührt, bevor man sie innerhalb von 10 Minuten auf Raumtemperatur erwärmte. Die so erhaltene Lösung wurde am Rotationsverdampfer bei einem Vakuum von 8 mbar vom Lösungsmittel befreit, wobei 175,1 g eines farblosen, hochviskosen Öles anfielen.

Dieses wurde in 157,1 g (1,48 Mol) Trimethylorthoformiat gelöst, bevor man unter Rühren 0,36 g konzentrierte Schwefelsäure zusetzte und die Mischung für 2 Stunden auf 50°C erwärmte. Anschließend wurden innerhalb von 1 Stunde 98,0 g Cyclohexanon (1,0 Mol) zugetropft. Man ließ 1,5 Stunden bei 50°C nachreagieren und destillierte die gebildeten Leichtsieder bei einer Sumpftemperatur von maximal 60°C zunächst im Wasserstrahlvakuum, dann bei 2,5 mbar vollständig ab.

Es verblieben 221,3 g eines hellgelben, hochviskosen Öles, das nach NMR-spektroskopischer Untersuchung (80 MHz) etwa 30 % Zielprodukt enthielt.

### Beispiel 10

### 2-Isopropyl-1,3-dioxan-5-carbonsäureethylester-5-cyano

Zu einer bei 0°C bis 10°C gerührten Mischung aus 62,0 g p-Formaldehyd (2,1 Mol), 200 g Ethanol und 0,25 g Natriumethanolat wurden innerhalb von 2,0 Stunden 113,0 g Cyanessigsäureethylester (1,0 Mol) dosiert. Die Reaktionsmischung wurde anschließend noch 1 Stunde bei 10°C gerührt, bevor man sie innerhalb von 10 Minuten auf Raumtemperatur erwärmte. Die so erhaltene Lösung wurde am Rotationsverdampfer bei einem Vakuum von 8 mbar vom Lösungsmittel befreit, wobei 175,1 g eines farblosen, hochviskosen Öles anfielen.

Dieses wurde in 157,1 g (1,48 Mol) Trimethylorthoformiat gelöst, bevor man unter Rühren 0,36 g konzentrierte Schwefelsäure zusetzte und die Mischung für 2 Stunden auf 50°C erwärmte. Anschließend wurden innerhalb von 0,75 Stunden 123,5 g Isobutyraldehyd (1,7 Mol) zugetropft. Man ließ 0,5 Stunden bei 50°C nachreagieren und destillierte die gebildeten Leichtsieder bei einer Sumpftemperatur von maximal 60°C zunächst im Wasserstrahlvakuum, dann bei 2,5 mbar vollständig ab.

Es verblieben 239,1 g eines hellgelben, hochviskosen Öles, das nach NMR-spektroskopischer Untersuchung (80 MHz) etwa 30 % Zielprodukt enthielt.

## Patentansprüche

1. Verfahren zur Herstellung von 1,3-Dioxanverbindungen der allgemeinen Formel 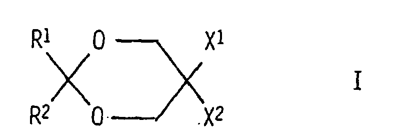 in der R¹ und R² jeweils unabhängig voneinander Wasserstoff, Alkyl, Aralkyl, Aryl oder Cycloalkyl mit jeweils bis zu 12 Kohlenstoffatomen bedeuten und zudem gemeinsam einen Alkylenrest mit 4 bis 11 Kohlenstoffatomen bezeichnen können und X¹ und X² jeweils unabhängig voneinander COOR, CONR₂, CN, NO₂, C(OR)=NR₂ oder COR bedeuten, wobei R für Wasserstoff, Alkyl, Aralkyl, Aryl oder Cycloalkyl mit jeweils bis zu 12 Kohlenstoffatomen steht; mit der Maßgabe, daß (i) nicht beide Substituenten X¹ und X² gleichzeitig COOH bedeuten können und (ii), wenn beide Substituenten X¹ und X² gleichzeitig COR bedeuten, die beiden Substituenten R auch einen Alkylenrest mit 2 bis 9 Kohlenstoffatomen bedeuten können,
**dadurch gekennzeichnet,**
**daß** man eine Bishydroxymethylverbindung der allgemeinen Formel 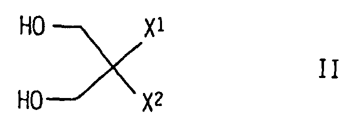 in der X¹ und X² jeweils unabhängig voneinander die angegebene Bedeutung haben, mit einem Orthocarbonsäureester der allgemeinen Formel
R⁴-C(OR⁵)₃ III
in der R⁴ Wasserstoff oder einen Kohlenwasserstoffrest und R⁵ einen Kohlenwasserstoffrest bedeuten, und mit einem Aldehyd oder Keton der allgemeinen Formel
R¹-CO-R² IV
umsetzt, in der R¹ und R² jeweils unabhängig voneinander die zuvor angegebene Bedeutung haben.

2. Verfahren nach Anspruch 1, **dadurch gekennzeichnet, daß** X¹ und X² jeweils unabhängig CN oder COOR' bedeuten, wobei R' einen C₁₋₄-Alkylrest bezeichnet.

3. Verfahren nach einem der Ansprüche 1 bis 2, **dadurch gekennzeichnet, daß** die Bishydroxymethylverbindung II nacheinander mit dem Orthocarbonsäureester III und dem Aldehyd oder Keton IV umgesetzt wird.

4. Verfahren nach einem der Ansprüche 1 bis 2, **dadurch gekennzeichnet, daß** die Umsetzung der Bishydroxymethylverbindung II mit dem Aldehyd oder Keton IV in Gegenwart eines sauren Katalysators erfolgt.

5. Verfahren nach Anspruch 4, **dadurch gekennzeichnet, daß** der saure Katalysator ein Alkalihydrogensulfat, gegebenenfalls zusammen mit Schwefelsäure, oder ein saurer Festbettkatalysator ist.

6. Verfahren nach einem der Ansprüche 1 bis 5, **dadurch gekennzeichnet, daß** man die Umsetzung absatzweise bei 0 bis 80°C oder bei 20 bis 50°C oder kontinuierlich bei einer Temperatur bis zu 200°C durchführt, wobei die Reaktionszeit der Reaktionstemperatur entsprechend so gewählt wird, daß praktisch keine Zersetzung stattfindet.

7. Verfahren nach Anspruch 6, **dadurch gekennzeichnet, daß** man bei der absatzweisen Herstellung die Reaktionstemperatur gegen Ende der Reaktion erhöht.

8. Verfahren nach einem der Ansprüche 1 bis 7, **dadurch gekennzeichnet, daß** während der Reaktion Leichtsieder aus dem Reaktionsgemisch abdestilliert werden.

9. Verfahren nach einem der Ansprüche 1 bis 8, **dadurch gekennzeichnet, daß** die Isolierung der 1,3-Dioxanverbindungen I durch fraktionierte Destillation des von Leichtsiedern befreiten Reaktionsgemisches erfolgt.

10. Verfahren nach einem der Ansprüche 1 bis 9, **dadurch gekennzeichnet, daß** die Bishydroxyxmethylverbindung II durch Umsetzung einer C-H-aciden Verbindungen der allgemeinen Formel 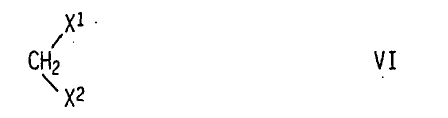 in der X¹ und X² die angegebene Bedeutung haben, mit Formaldehyd oder einer Formaldehyd abgebenden Verbindung hergestellt und ohne Isolierung im Reaktionsgemisch umgesetzt wird.

11. Verfahren nach Anspruch 10, **dadurch gekennzeichnet, daß** die Umsetzung in einem Orthocarbonsäureester als Lösungsmittel durchgeführt wird.

## Claims

1. A method of preparingal, 3-dioxane compounds of the general formula 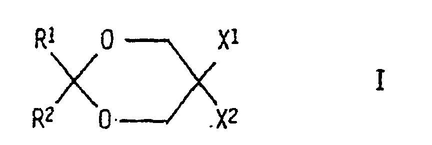 in which R¹ and R² each, independently of one another, represent hydrogen, alkyl, aralkyl, aryl or cycloalkyl, each having up to 12 carbon atoms, and may also jointly denote an alkylene radical having from 4 to 11 carbon atoms; and X¹ and X² each, independently of one another, represent COOR, CONR₂, CN, NO₂, C(OR)=NR₂ or COR, R representing hydrogen, alkyl, aralkyl, aryl or cycloalkyl, each having up to 12 carbon atoms; with the proviso that (i) the substituents X¹ and X² cannot both simultaneously represent COOH and (ii) if the two substituents X¹ and X² simultaneously represent COR, the two substituents R may also represent an alkylene radical having from 2 to 9 carbon atoms, **characterized in that** a bis(hydroxymethyl) compound of the general formula 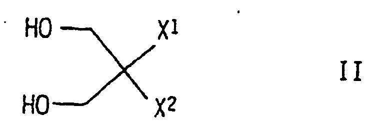 in which X¹ and X² each, independently of one another, have the specified meanings, is reacted with an orthocarboxylic acid ester of the general formula
R⁴-C(OR⁵)₃ III
in which R⁴ represents hydrogen or a hydrocarbon radical and R⁵ represents a hydrocarbon radical, and with an aldehyde or ketone of the general formula
R¹-CO-R² IV
in which R¹ and R² each, independently of one another, have the above-specified meanings.

2. A method according to claim 1, **characterized in that** X¹ and X² each independently represent CN or COOR', R' denoting a C₁₋₄-alkyl radical.

3. A method according to any one of claims 1 to 2, **characterized in that** the bis(hydroxymethyl) compound II is successively reacted with the orthocarboxylic acid ester III and the aldehyde or ketone IV.

4. A method according to any one of claims 1 to 2, **characterized in that** the reaction of the bis(hydroxymethyl) compound II with the aldehyde or ketone IV is carried out in the presence of an acidic catalyst.

5. A method according to claim 4, **characterized in that** the acidic catalyst is an alkali metal hydrogen sulphate, if desired together with sulphuric acid, or an acidic fixed-bed catalyst.

6. A method according to any one of claims 1 to 5, **characterized in that** the reaction is carried out batchwise at from 0 to 80°C or at from 20 to 50°C or continuously at a temperature up to 200°C, the reaction time being selected in accordance with the reaction temperature so as to ensure that virtually no decomposition takes place.

7. A method according to claim 6, **characterized in that** in the case of the batchwise preparation the reaction temperature is raised toward the end of the reaction.

8. A method according to any one of claims 1 to 7, **characterized in that** low-boiling components are distilled off during the reaction from the reaction mixture.

9. A method according to any one of claims 1 to 8, **characterized in that** the 1,3-dioxane compounds I are isolated by fractional distillation of the reaction mixture freed from low-boiling components.

10. A method according to any one of claims 1 to 9, **characterized in that** the bis(hydroxymethyl) compound II is prepared by reacting a C-H-acidic compound of the general formula 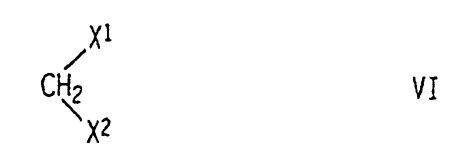 in which X¹ and X² have the specified meanings, with formaldehyde or a formaldehyde-releasing compound, and without isolation is reacted in the reaction mixture.

11. A method according to claim 10, **characterized in that** the reaction is carried out in an orthocarboxylic acid ester as the solvent.

## Revendications

1. Procédé pour la préparation de composés de 1,3-dioxane de formule générale : 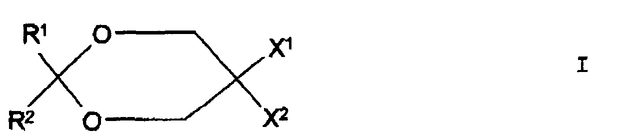 dans laquelle R¹ et R², respectivement et indépendamment l'un de l'autre, désignent l'hydrogène, un groupe alkyle, aralkyle, aryle ou cycloalkyle ayant respectivement jusqu'à 12 atomes de carbone et peuvent en outre désigner ensemble un radical alkyle ayant 4 à 11 atomes de carbone, et X¹ et X² respectivement et indépendamment l'un de l'autre, désignent COOR, CONR₂, CN, NO₂, C(OR)=NR₂ ou COR, R étant l'hydrogène, un groupe alkyle, aralkyle, aryle ou cycloalkyle ayant respectivement jusqu'à 12 atomes de carbone, à condition que (i) les deux substituants X¹ et X² ne peuvent pas désigner en même temps COOH et (ii), si les deux substituants X¹ et X² désignent en même temps COR, les deux substituants R peuvent également désigner un radical alkyle ayant 2 à 9 atomes de carbone,
**caractérisé en ce que**
l'on transforme un composé de bishydroxyméthyle de formule générale : 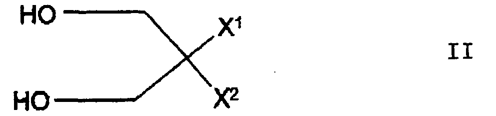 dans laquelle X¹ et X², respectivement et indépendamment l'un de l'autre, ont la signification donnée, avec un ester d'acide orthocarbonique de formule générale :
R⁴-C(OR⁵)₃ III
dans laquelle R⁴ désigne l'hydrogène ou un radical hydrocarboné et R⁵, un radical hydrocarboné et est transformé avec un aldéhyde ou une cétone de formule générale :
R¹-CO-R² IV
dans laquelle R¹ et R², respectivement indépendamment l'un de l'autre, ont la signification donnée auparavant.

2. Procédé selon la revendication 1,
**caractérisé en ce que**
X¹ et X², respectivement et indépendamment, désignent CN ou COOR', R' désignant un radical alkyle en C₁-C₄.

3. Procédé selon l'une des revendications 1 à 2,
**caractérisé en ce que**
le composé de bishydroxyméthyle II est transformé, dans l'ordre, avec l'ester d'acide orthocarbonique III et avec l'aldéhyde ou la cétone IV.

4. Procédé selon l'une des revendications 1 à 2,
**caractérisé en ce que**
la transformation du composé de bishydroxyméthyle II se produit avec l'aldéhyde ou la cétone IV en présence d'un catalyseur acide.

5. Procédé selon la revendication 4,
**caractérisé en ce que**
le catalyseur acide est un hydrogénosulfate alcalin, le cas échéant associé à de l'acide sulfurique ou un catalyseur à lit fixe acide.

6. Procédé selon l'une des revendications 1 à 5,
**caractérisé en ce que**
l'on réalise la transformation par paliers à 0-80 °C ou à 20-50 °C ou en continu à une température allant jusqu'à 200 °C, le temps de réaction de la température réactionnelle correspondante étant choisi de telle sorte qu'il ne se produit pratiquement aucune décomposition.

7. Procédé selon la revendication 6,
**caractérisé en ce que**
lors de la préparation par paliers, on élève la température réactionnelle vers la fin de la réaction.

8. Procédé selon l'une des revendications 1 à 7,
**caractérisé en ce que**
lors de la réaction, des produits à bas point d'ébullition sont séparés par distillation du mélange réactionnel.

9. Procédé selon l'une des revendications 1 à 8,
**caractérisé en ce que** l'isolement des composés de 1,3-dioxane I se produit par distillation fractionnée du mélange réactionnel débarrassé des produits à bas point d'ébullition.

10. Procédé selon l'une des revendications 1 à 9,
**caractérisé en ce que**
le composé de bishydroxyméthyle II est préparé par transformation d'un composé acide C-H de formule générale : 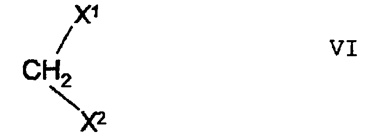 dans laquelle X¹ et X² ont la signification donnée, avec du formaldéhyde ou un composé dégageant du formaldéhyde et transformé sans isolement dans le mélange réactionnel.

11. Procédé selon la revendication 10,
**caractérisé en ce que**
la transformation se produit dans un ester d'acide orthocarbonique en tant que solvant.
